# FASCICULE DE BREVET EUROPEEN

(11) **EP 3 052 154 B1**
(45) Date de publication et mention de la délivrance du brevet: **26.01.2022**
(21) Numéro de dépôt: 14780841.4
(22) Date de dépôt: 02.10.2014
(51) Int. Cl.: A61L 27/36

(54) **MATERIAU DE REGENERATION OSSEUSE ET SON PROCEDE DE FABRICATION**
KNOCHENREGENERIERUNGSMATERIAL UND HERSTELLUNGSVERFAHREN DAFÜR
BONE REGENERATION MATERIAL AND MANUFACTURE METHOD THEREOF

(30) Priorité: 03.10.2013 BE 201300660
(43) Date de publication de la demande: 10.08.2016
(73) Titulaire: Wishbone, 4460 Grâce-Hollogne (BE)
(72) Inventeur: ROMPEN, Eric, 4053 Embourg (BE); LAMBERT, France, 4000 Liège (BE); LECLOUX, Geoffrey, 4050 Chaudfontaine (BE); MONIOTTE, Philippe, 4217 Héron (BE)
(74) Mandataire: Calysta NV
(86) Numéro de dépôt international: PCT/EP2014/071157
(87) Numéro de publication internationale: WO 2015/049336

(56) Documents cités:
- WO-A1-96/12509
- US-A- 5 417 975
- US-A1- 2003 014 124

## Description

La présente invention se rapporte à un procédé de fabrication d'un matériau de régénération osseuse comprenant de l'hydroxyapatite.

Ce type de matériau de régénération osseuse est en particulier utilisé dans le cadre du traitement de détériorations osseuses et ce, dans différent domaines de la chirurgie réparatrice ou esthétique.

L'hydroxyapatite est un phosphate de calcium aux propriétés ostéoconductrices et constitue la composante minérale principale de l'os, et son utilisation convient donc parfaitement dans le cadre d'une chirurgie reconstructrice ou réparatrice de l'os.

C'est dans ce cadre que de nombreux matériaux comprenant de l'hydroxyapatite sont actuellement utilisés comme implants, et en particulier comme implants dentaires, pour stimuler la reconstruction osseuse sur des sites osseux présentant des défauts ou des détériorations.

Parmi ces matériaux, on compte les matériaux artificiels comprenant de l'hydroxyapatite qui est préparée par synthèse à partir d'acide phosphorique et de sels de calcium, soit par un procédé de précipitation directe, soit par un procédé de type sol-gel.

Cependant, les procédés de synthèse susmentionnés ne permettent pas d'obtenir un matériau de régénération osseuse dans lequel l'hydroxyapatite présente une structure spatiale poreuse similaire à celle que l'on retrouve dans une matrice osseuse à l'état naturel.

C'est la raison pour laquelle, actuellement, il existe un intérêt grandissant pour l'utilisation d'une hydroxyapatite d'origine naturelle dont l'avantage est de présenter une structure cristalline et une morphologie identiques à celles d'un matériau osseux à l'état naturel.

En particulier, l'utilisation d'une hydroxyapatite naturelle provenant de échantillons osseux, une fois nettoyés de leurs substances organiques comprenant au moins un composant du groupe constitué des protéines, comme des prions, des peptides, et des lipides, permet d'obtenir une matrice plus propice à la régénération osseuse que son analogue artificielle.

Il est en effet impératif que le matériau de régénération osseuse, lorsqu'il est par exemple à l'état implanté, soit épuré de toute trace de substances organiques de sorte que, d'une part, ce dernier, une fois à l'état implanté, soit bien intégré par l'organisme, soit biocompatible et interagisse avec l'environnement biologique dans lequel il est placé par ostéoconduction.

Dans ce contexte, la présente invention se rapporte à un procédé de fabrication d'un matériau de régénération osseuse comprenant:
- une mise en contact d'un matériau osseux contenant de l'hydroxyapatite et des substances organiques avec un liquide d'extraction qui donne lieu à une phase liquide contenant lesdites substances organiques et éventuellement des impuretés extraites dudit matériau osseux et à une phase hydroxyapatite solide contenant ladite hydroxyapatite, et
- une séparation entre ladite phase liquide et ladite phase hydroxyapatite solide.

Un tel procédé est connu par exemple des documents US5417975 et WO96/12509. Plus particulièrement, le document US5417975 divulgue la fabrication d'un matériau de régénération osseuse d'origine naturelle bien connu qui est le Bio-Oss^{®} et le document WO96/12509 divulgue la préparation d'un matérieu ostéoinductif.

A côté des propriétés ostéoconductrices améliorées d'un matériau de régénération osseuse d'origine naturelle, la production de ce dernier permet en outre de valoriser des déchets organiques d'abattoir et de boucherie qui constituent une source bon marché et abondante en matériaux osseux.

Dans le procédé selon US5417975, le matériau osseux est d'abord dégraissé pour ensuite être placé dans une solution aqueuse chauffée, de préférence à une température comprise entre 80 et 200°C, d'amines primaires ou d'ammoniac dans lequel les substances organiques sont dégradées et solubilisées pour enfin être extraites par des rinçages successifs à l'eau déminéralisée, de préférence chauffée à une température comprise entre 20 et 60°C, à une vitesse de passage avantageusement d'au moins 10 cm par heure.

Le dégraissage est réalisé par trempage du matériau osseux dans un solvant organique, tel que le toluène ou le méthylcyclohexane, chauffé en reflux à une température comprise entre 80 et 120°C.

Selon US5417975, la période durant laquelle le matériau osseux doit être mis en contact avec ladite solution aqueuse chauffée dépend de la granulométrie du matériau, de la réactivité des amines primaires ou de la solution d'ammoniac, et de la température à laquelle est chauffée cette solution aqueuse. Généralement, cette période est comprise entre 2 et 200 heures. A titre d'exemple, un échantillon de matériau osseux de 1 cm de diamètre traité par une solution aqueuse d'éthylène diamine à 118°C nécessite un temps de traitement d'au moins 50 heures pour obtenir une dégradation satisfaisante des substances organiques de sorte que, après rinçage, elles restent présentes dans une teneur inférieure à 150 ppm.

Le matériau osseux ainsi traité est ensuite séché à l'air sous des températures comprises entre 250°C et 600°C jusqu'à ce que le poids en matériau se stabilise à une valeur constante. Afin d'obtenir un matériau de régénération osseuse de grande pureté, c'est-à-dire dans lequel la teneur en substances organiques est inférieure à 150 ppm, l'étape de rinçage à l'eau déminéralisée doit être réalisé en continu sur une période allant de 5 à 25 jours.

Malheureusement, un tel procédé souffre de plusieurs limitations. D'abord, il implique un traitement utilisant des solvants organiques dont les résidus, après traitement, ne sont pas valorisables et doivent être traités. Ensuite, ce procédé implique un traitement dans un bain d'amines à 60°C pendant plus de 50 heures et suivi d'un rinçage à l'eau déminéralisée en flux continu sur une durée d'au moins 5 jours. Clairement, la durée importante du procédé (au moins 5 jours) impliquant de surcroît l'utilisation de produits organiques polluants qui doivent être traités rend la mise à l'échelle industrielle d'un tel procédé onéreuse et difficilement amortissable.

L'invention a pour but de pallier les inconvénients de l'état de la technique en procurant un procédé permettant la fabrication plus rapide d'un matériau de régénération osseuse exempt de toute trace de substances organiques indésirables, dont la mise en œuvre est plus simple, dont l'impact négatif de ses étapes sur l'environnement est limité, et dont les sous-produits de fabrication sont facilement traitables.

En outre, l'invention a pour but de proposer un procédé de fabrication d'un matériau de régénération osseuse dans lequel toutes les étapes peuvent être réalisées à l'échelle industrielle de façon continue ou semi-continue dans un réacteur unique. Cette applicabilité industrielle est facilement réalisable dès lors que le procédé suivant l'invention est particulièrement efficace en termes de rendement, en termes de consommation énergétique et qu'il n'utilise que des réactifs relativement peu coûteux tels que de l'eau et de la soude, ce qui permet par ailleurs de disposer d'un procédé « vert », c'est-à-dire d'un procédé respectueux de l'environnent.

Pratiquement, le procédé doit permettre la fabrication d'un matériau de régénération osseuse ainsi obtenu par procédé selon l'invention comprend des substances organiques à une teneur inférieure à 150 ppm.

Avantageusement, le procédé doit permettre la fabrication d'un matériau de régénération osseuse comprenant des protéines à une teneur inférieure à 130 ppm, et une absence complète de protéines ou de peptides intacts, en particulier des prions.

Pour résoudre ce problème, il est prévu suivant l'invention, un procédé tel qu'indiqué au début caractérisé en ce que ledit liquide d'extraction est une solution aqueuse d'extraction amenée à une température comprise entre 150°C et 300°C et à une pression comprise entre 1500 kPa et 3500 kPa, et en ce que ladite phase hydroxyapatite solide séparée forme ledit matériau de régénération osseuse.

En effet, il est observé de manière tout à fait surprenante que la mise en contact du matériau osseux avec la solution aqueuse d'extraction à des conditions dites « intensifiées », c'est-à-dire à une température comprise entre 150°C et 300°C et à une pression comprise entre 1500 kPa et 3500 kPa, avec le matériau osseux permet la production d'une phase hydroxyapatite solide comprenant de manière exclusive de l'hydroxyapatite formant le matériau osseux, grâce à une extraction dans la phase liquide de l'ensemble des substances organiques indésirables ainsi que d'autres impuretés indésirables qui, si elles étaient présentes dans la phase solide, compromettraient la biocompatibilité de l'hydroxyapatite et augmenterait les chances de rejet lors de sa pose en tant qu'implant osseux. Les caractéristiques de l'eau aux conditions intensifiées sont telles que celle-ci se comporte à la fois comme à la fois un solvant et un réactif. La température élevée, c'est-à-dire comprise entre 150°C et 300°C, utilisée dans le procédé a pour effet d'accroître la dissociation de l'eau (et donc sa réactivité), d'accélérer toutes les réactions, en particuliers d'hydrolyse, et de diminuer notablement la viscosité du solvant. Cette dernière propriété étant avantageuse dans le cas du traitement d'un solide poreux insoluble par un réactif liquide, où la vitesse de diffusion du liquide dans la matrice solide conditionne l'efficacité et la vitesse des échanges chimiques.

Dans ce contexte, de façon tout à fait inattendue, il a été montré, dans le cadre de la présente invention, que les étapes d'extraction et de rinçage réalisées sous les conditions de températures et de pressions selon l'invention ne requièrent qu'une durée de l'ordre de 2 à 5 heures, au contraire de l'état de la technique et notamment du document US5417975 où cette durée est de près de 50 heures, soit dix fois supérieure. Plus spécifiquement, suivant l'invention, l'étape d'extraction elle-même est inférieure à 1 heure, ce qui limite fortement la consommation énergétique et rend le procédé selon l'invention d'autant plus performant et applicable industriellement.

En outre, ces conditions de températures et de pressions permettent la solubilisation de composés organiques qui sont connus pour être insolubles dans l'eau sous les conditions normales de température et de pression. De plus, l'hydroxyapatite reste insoluble dans la phase aqueuse sous ces conditions intensifiées.

Eventuellement, préalablement à ladite étape de séchage, ladite première étape de mise en contact et ladite étape de séparation sont réalisées plusieurs fois et de manière successive.

Avantageusement, le procédé suivant l'invention comprend en outre une étape additionnelle de séchage de ladite phase hydroxyapatite solide pour obtenir une phase hydroxyapatite solide sèche.

Avantageusement, le poids en phase hydroxyapatite solide correspond à une fraction comprise entre 55 et 65 %, de préférence entre 57 et 62 %, du poids dudit matériau osseux avant que celui-ci n'ait été traité par le procédé selon, l'invention.

De préférence, la mise en contact du matériau osseux avec la solution aqueuse d'extraction est réalisée à une température comprise entre 220 et 250°C, avantageusement inférieure à 240°C.

Il a en effet été observé dans le cadre de la présente invention que le procédé atteint un optimum d'efficacité à ces températures, c'est-à-dire que le procédé permet la fabrication d'un matériau de régénération osseuse dans lequel la teneur en protéines est strictement inférieure à 130 ppm, des températures plus élevées induisant une décomposition trop rapide des protéines comprises dans les substances organiques et leurs résidus initiaux d'hydrolyse, c'est-à-dire favorisant la formation dans la phase hydroxyapatite de résidus organiques solides insolubles qui ne peuvent être extraits par le liquide d'extraction et dont leur présence peut compromettre la biocompatibilité du matériau de régénération.

Alternativement, la mise en contact du matériau osseux avec la solution aqueuse d'extraction est réalisée à une pression comprise entre 2500 kPa et 3000 kPa.

Avantageusement, ladite solution aqueuse d'extraction est de l'eau ou une solution aqueuse à pH basique concentrée par exemple formée à partir d'une solubilisation dans de l'eau d'une base forte, de préférence un hydroxyde alcalin, à une concentration comprise entre 0,5 et 1 N, de préférence entre 0,6 et 0,9 N.

Il a été en outre observé que, à pH basique, l'extraction des substances organiques, telles que les résidus d'acides gras généralement peu soluble dans l'eau, est améliorée.

En outre, à un pH supérieur à 7 la solubilité de l'hydroxyapatite reste très faible. Dès lors, en réalisant l'étape d'extraction à un pH basique, on assure l'insolubilité de l'hydroxyapatite dans le liquide d'extraction.

Dans un mode particulier de réalisation du procédé selon l'invention, ladite étape de séchage est une étape de séchage sous étuve à chaleur sèche, à une température comprise entre 100°C et 300°C, de préférence à 120°C.

De préférence, après ladite première étape de séparation et avant ladite étape de séchage de ladite phase hydroxyapatite solide, le procédé selon l'invention comprend une deuxième étape de mise en contact avec une solution aqueuse à pH basique diluée de ladite phase hydroxyapatite solide à une température comprise entre 150°C et 300°C, de préférence à 150°C, et à une pression comprise entre 99 kPa et 1000 kPa, de préférence à 500 kPa, ladite deuxième étape de mise en contact étant suivie par une deuxième étape de séparation entre une deuxième phase liquide et une deuxième phase solide pour ensuite être séchée.

Avantageusement, ladite phase aqueuse à pH basique diluée est formée à partir d'une solubilisation dans de l'eau d'une base forte, de préférence un hydroxyde alcalin, à une concentration comprise entre 0,05 et 0,4 N, de préférence entre 0,1 et 0,3 N.

Eventuellement, ladite phase hydroxyapatite solide est préalablement rincée à l'eau avant d'être séchée.

Dans un mode préférentiel de réalisation, le procédé selon l'invention est caractérisé en ce qu'il comprend, préalablement à la première étape de mise en contact avec ledit liquide d'extraction, une étape de dégraissage et de décharnage dudit matériau osseux.

De préférence, cette étape de dégraissage et de décharnage est réalisée en mettant en contact ledit matériau osseux à une solution aqueuse, de préférence à pH basique, à une température supérieure ou égale à 100°C, de préférence comprise entre 100 et 200°C.

De manière préférentielle, l'étape de dégraissage et de décharnage est suivie d'une étape de grattage des résidus de chair, de moelle et de cartilage présents sur le matériau osseux.

Avantageusement, le procédé selon l'invention comprend, postérieurement à ladite étape de séchage, une étape de stérilisation de ladite phase hydroxyapatite solide sèche.

Dans un mode particulier de réalisation du procédé selon l'invention, ladite étape de stérilisation est réalisée par exemple, par chauffage à 120°C durant 14 heures de ladite phase hydroxyapatite sèche dans un sac en fluoropolymère étanche à l'air. De préférence, la température de séchage est comprise entre 100°C et 300°C durant une période comprise entre 5 et 20 heures, de préférence entre 10 et 15 heures.

De manière préférentielle, ledit matériau osseux est d'origine naturelle animale, par exemple d'un mammifère ou d'un ovipare, et peut être d'origine bovine, ovine, équine, ou encore porcine, et analogues, en particulier extrait de l'épiphyse ou de la métaphyse de ces animaux..

De manière générale, le matériau osseux provient de déchets de boucherie de sorte que le procédé selon l'invention présente l'avantage de valoriser ces déchets disponibles de préférence en abondance.

D'autres formes de réalisation de la composition suivant l'invention sont indiquées dans les revendications annexées.

D'autres caractéristiques et avantages de l'invention ressortiront de la description donnée ci-après, à titre non-limitatif et en faisant référence aux exemples comparatifs décrits ci-dessous.
La figure 1 illustre les spectres infrarouges (IR) superposés, d'une part, du matériau de régénération (a) obtenu par le procédé selon l'invention et, d'autre part, du Bio-Oss^{®} (b).
La figure 2 illustre les spectres IR superposés, d'une part, du matériau de régénération (a) obtenu par le procédé selon l'invention et, d'autre part, du matériau osseux (b) avant traitement par le procédé selon l'invention.
La figure 3 illustre les spectres IR superposés de matériau de régénération (a) obtenu par le procédé selon l'invention, du matériau osseux (b) avant traitement par le procédé selon l'invention, et d'une protéine de référence : la zéine (c).
La figure 4 illustre une image obtenue par microscopie à balayage électronique (MEB) de la structure du Bio-Oss^{®} à une échelle allant de 0 à 100 µm.
La figure 5 illustre deux images MEB prises à une échelle allant de 0 à 50 µm et à une échelle allant de 0 à 100 µm du matériau de régénération osseuse obtenu par le procédé selon l'invention à partir d'épiphyse de bœuf.
La figure 6 illustre deux images MEB prises à une échelle allant de 0 à 100 µm du matériau de régénération osseuse obtenu par le procédé selon l'invention à partir de métaphyse de veau.
La figure 7 illustre une image MEB prises à une échelle allant de 0 à 50 µm du matériau de régénération osseuse obtenu par le procédé selon l'invention à partir de métaphyse de bœuf.
La figure 8 illustre deux images MEB prises à une échelle allant de 0 à 100 µm du matériau de régénération osseuse obtenu, à partir d'épiphyse de bœuf, par un procédé dans lequel le matériau osseux a été calciné à 800°C.

Les exemples qui suivent ont pour but d'illustrer de manière non limitative la présente invention.

Dans les exemples qui suivent, le matériau osseux a été préalablement dégraissé et décharné par ébullition prolongée dans de l'eau suivie d'un grattage pour enlever les résidus de chair, de moelle et de cartilage.

### Exemple 1

50g d'un matériau osseux, dégraissé et décharné, contenant de l'hydroxyapatite, 400ml d'eau déminéralisée et 10g d'une base forte (NaOH) sont mélangés dans un réacteur d'autoclave sans agitateur amené à une température de 220°C ± 2°C par chauffage externe au moyen d'un manteau électrique. La pression dans le réacteur est amenée à 2500 kPa. Ces conditions de température et de pression sont maintenues pendant une durée d'une heure.

Le réacteur est refroidi pour être amené à température ambiante et dépressurisé à la pression atmosphérique, une phase liquide homogène est éliminée du réacteur par décantation pour qu'il ne reste plus qu'une phase solide dans le réacteur à laquelle on ajoute 5g de NaOH en solution dans 400ml d'eau déminéralisée.

Le réacteur de l'autoclave est ensuite amené à une température de 150°C sous une pression atmosphérique (environ 100 kPa) pendant une durée d'une heure.

Le réacteur est à nouveau refroidi pour être amené à température ambiante, le contenu du réacteur est décanté, et la phase solide, qui est une phase hydroxyapatite solide, est rincée à l'eau déminéralisée pour être ensuite séchée sous étuve à une température de 120°C jusqu'à ce que son poids se stabilise vers une valeur stationnaire.

La phase hydroxyapatite solide forme le matériau de régénération osseuse dont le spectre IR est illustré à la figure 1 et comparé à celui du Bio-Oss^{®}. Les spectres IR sont identiques, ce qui indique que le matériau de régénération osseuse obtenu par le procédé selon l'invention est identique au Bio-Oss^{®}.

La figure 2 illustre la superposition entre, d'une part, le spectre IR du matériau de régénération (a) obtenu par le procédé selon l'invention et, d'autre part, le spectre IR du matériau osseux (b) avant traitement par le procédé selon l'invention.

Cette superposition permet de voir la contribution des protéines présentes dans le matériau osseux à la composition du spectre IR du matériau de régénération selon l'invention. Cette contribution est principalement caractérisée par des bandes d'absorption très intenses entre 3500 et 3000 cm⁻¹ et entre 1750 et 1500 cm⁻¹ qui ne sont plus présentes dans le matériau de régénération obtenu par le procédé selon l'invention.

La figure 3 illustre par ailleurs la comparaison entre le spectre IR du matériau osseux décharné et dégraissé et celui d'une protéine de référence : la zéine. La correspondance entre les pics spécifiques dans les régions du spectre électromagnétique situées entre 3500 et 3000 cm⁻¹ et entre 1750 et 1500 cm⁻¹ confirme la présence quasi-exclusive de protéines dans le matériau osseux après dégraissage et décharnage de ce dernier.

La figure 4 est une image MEB de la structure du Bio-Oss^{®} confirmant la structure macroporeuse de celui-ci. A la lecture de cette figure, il apparait clairement que ce matériau présente une structure comprenant deux types de pores : des pores de diamètre d'environ 50 µm correspondant à l'extraction des protéines, et des pores de diamètre d'environ 100 µm inhérents à la structure du matériau osseux.

Les figures 5 à 7 illustre les images MEB de la structure du matériau de régénération osseuse obtenu respectivement à partir d'épiphyse de bœuf (figure 5), de métaphyse de veau (figure 6), et de métaphyse de bœuf (figure 7).

Parmi les différents matériaux de régénération osseuse préparés selon l'exemple 1, c'est celui obtenu à partir de métaphyse de veau qui présente une structure macroporeuse similaire à celle du Bio-Oss^{®}.

### Exemple 2

100g d'un matériau osseux, dégraissé et décharné, contenant de l'hydroxyapatite, 400ml d'eau déminéralisée et 10g de NaOH sont mélangés dans un réacteur d'autoclave sans agitateur amené à une température de 230°C ± 2°C par chauffage externe au moyen d'un manteau électrique. La pression dans le réacteur est amenée à 2500 kPa. Ces conditions de température et de pression sont maintenues pendant une durée de deux heures.

Le réacteur est refroidi pour être amené à température ambiante et dépressurisé à la pression atmosphérique, une phase liquide homogène est éliminée du réacteur par décantation pour qu'il ne reste plus qu'une phase solide dans le réacteur à laquelle on ajoute 3g de NaOH en solution dans 400ml d'eau déminéralisée.

Le réacteur de l'autoclave est ensuite amené à une température de 150°C sous une pression atmosphérique (environ 100 kPa) pendant une durée de deux heures.

Le réacteur est à nouveau refroidi pour être amené à température ambiante, le contenu du réacteur est décanté et la phase hydroyapatite solide est rincée cinq fois à l'eau déminéralisée pour être ensuite séchée sous étuve à une température de 120°C jusqu'à ce que son poids se stabilise vers une valeur stationnaire.

La phase hydroxyapatite solide ainsi obtenue est broyée puis tamisée en deux fractions de 1,0-0,25mm et de 0,25-0,08mm, pour être ensuite stérilisée à 120°C dans des sacs en fluoropolymère étanches à l'aire pendant une période de 14 heures.

### Exemple 3

100g d'épiphyse de bœuf, dégraissé et décharné, contenant de l'hydroxyapatite, 400ml d'eau déminéralisée et 15g de NaOH, sont mélangés dans un réacteur d'autoclave sans agitateur amené à une température de 230°C ± 2°C par chauffage externe au moyen d'un manteau électrique. La pression dans le réacteur est amenée à 2500 kPa. Ces conditions de température et de pression sont maintenues pendant une durée de deux heures.

Le réacteur est refroidi pour être amené à température ambiante et dépressurisé à la pression atmosphérique, une première phase liquide homogène est éliminée du réacteur par décantation pour qu'il ne reste plus qu'une phase solide dans le réacteur qui est rincée à l'eau déminéralisée.

La phase solide est à nouveau mélangée à 400ml d'eau déminéralisée et 10g de NaOH pour ensuite être soumise une nouvelle fois au traitement susmentionné.

Une deuxième phase liquide homogène est éliminée du réacteur par décantation pour qu'il ne reste plus qu'une phase solide dans le réacteur à laquelle on ajoute 1g de NaOH en solution dans 400ml d'eau déminéralisée. Le réacteur de l'autoclave est ensuite amené à une température de 150°C sous une pression atmosphérique (environ 100 kPa) pendant une durée de deux heures.

Ensuite, le réacteur est à nouveau refroidi pour être amené à température ambiante, le contenu du réacteur est décanté et la phase hydroyapatite solide est rincée cinq fois à l'eau déminéralisée pour être ensuite séchée sous étuve à une température de 120°C jusqu'à ce que son poids se stabilise vers une valeur stationnaire.

La phase hydroxyapatite solide ainsi obtenue est broyée puis tamisée en une seule fraction de 1,0-0,25mm.

### Exemple 4

100g de matériau osseux sont calcinés dans un four à moufle à une température de 800°C ± 2°C pendant une heure jusqu'à blanchiment complet du matériau.

Après refroidissement, la phase hydroxyapatite solide ainsi obtenue est broyée puis tamisée en deux fractions de 1,0-0,25mm et de 0,25-0,08mm, pour être ensuite stérilisée à 120°C dans des sacs en fluoropolymère étanches à l'aire pendant une période de 14 heures.

La figure 8 illustre la structure macroscopique obtenue par imagerie MEB de la phase solide obtenue selon le protocole de l'exemple 4. Comme le montre cette figure, la calcination à 800°C du matériau osseux produit une phase hydroxyapatite solide dont la structure est différente de celle du Bio-Oss^{®} puisqu'on constate un début d'effondrement du réseau d'hydroxyapatite qui résulte en une diminution de la porosité de la structure et qui s'accompagne par ailleurs d'une apparition de craquelures de taille de l'ordre de 20 µm.

Les résultats de cet exemple tendent à démontrer que le traitement de l'hydroxyapatite à haute température détériore la morphologie et dont les propriétés ostéoconductrices du matériau de régénération osseuse.

En effet, une augmentation de la température jusqu'à 800°C montre qu'il y a une réduction des volumes poreux de la structure de l'hydroxyapatite, réduction probablement due à un début de frittage.

### Exemple 5

Dans le mode de réalisation décrit dans le présent exemple et contrairement aux modes de réalisations décrits dans les exemples précédents, un premier nettoyage de 1,7Kg d'épiphyse de veau est réalisé par deux étapes successives d'ébullition durant deux heures dans trois litres d'eau dans laquelle est dissout 5g de NaOH pour former une solution aqueuse basique qui est renouvelée à chaque étape.

Après chaque étape d'ébullition, un grattage et réalisé pour enlever les résidus de chair, de moelle, et de cartilage.

Le matériau osseux ainsi traité est découpé sur une épaisseur de 5mm. 100g de ce matériau, 400ml d'eau déminéralisée et 10g de NaOH sont mélangés dans un réacteur d'autoclave sans agitateur amené à une température de 230°C ± 2°C par chauffage externe au moyen d'un manteau électrique. La pression dans le réacteur est amenée à 2500 kPa. Ces conditions de température et de pression sont maintenues pendant une durée de deux heures.

Le réacteur est refroidi pour être amené à une température de 50°C et dépressurisé à la pression atmosphérique, une phase liquide homogène est éliminée du réacteur par décantation pour qu'il ne reste plus qu'une phase solide dans le réacteur à laquelle on ajoute 3g de NaOH en solution dans 400ml d'eau déminéralisée.

Le réacteur de l'autoclave est ensuite amené à une température de 150°C sous une pression atmosphérique (environ 100 kPa) pendant une durée de deux heures.

Le réacteur est à nouveau refroidi pour être amené à température ambiante, le contenu du réacteur est décanté et la phase hydroyapatite solide est rincée cinq fois à l'eau déminéralisée pour être ensuite séchée sous étuve à une température de 120°C jusqu'à ce que son poids se stabilise vers une valeur stationnaire.

La phase hydroxyapatite solide ainsi obtenue est broyée puis tamisée en trois fractions de 2,0-1,0mm, 1,0-0,25mm et inférieure à 0,25mm.

Il doit être entendu que la présente invention n'est en aucune façon limitée aux modes de réalisation décrits ci-dessus et que bien des modifications peuvent y être apportées dans le cadre des revendications annexées.

## Revendications

1. Procédé de fabrication d'un matériau de régénération osseuse comprenant:
- une mise en contact d'un matériau osseux contenant de l'hydroxyapatite et des substances organiques avec un liquide d'extraction qui donne lieu à une première phase liquide contenant lesdites substances organiques et éventuellement des impuretés extraites dudit matériau osseux et à une deuxième phase hydroxyapatite solide contenant ladite hydroxyapatite, et
- une séparation entre ladite phase liquide et ladite phase hydroxyapatite solide,
**caractérisé en ce que** ledit liquide d'extraction est une solution aqueuse d'extraction amenée à une température comprise entre 150°C et 300°C et à une pression comprise entre 1500 kPa et 3500 kPa, et **en ce que** ladite phase hydroxyapatite solide séparée forme ledit matériau de régénération osseuse.

2. Procédé selon la revendication 1, comprenant en outre une étape additionnelle de séchage de ladite phase hydroxyapatite solide pour obtenir une phase hydroxyapatite solide sèche.

3. Procédé selon la revendication 1 ou 2, dans lequel ladite solution aqueuse d'extraction est de l'eau ou une solution aqueuse à pH basique concentrée comprenant une base forte à une concentration comprise entre 0,5 et 1N, de préférence entre 0,6 et 0,9 N..

4. Procédé selon l'une quelconque de revendications précédentes, **caractérisé en ce que**, après ladite première étape de séparation et avant ladite étape de séchage de ladite phase hydroxyapatite solide, il comprend une deuxième étape de mise en contact avec une solution aqueuse à pH basique diluée de ladite phase hydroxyapatite solide à une température comprise entre 150°C et 300°C et à une pression comprise entre 500 kPa et 1000 kPa, ladite solution aqueuse à pH basique dilué comprenant une base forte à une concentration comprise entre 0,05 et 0,4 N, de préférence entre 0,1 et 0,3 N, ladite deuxième étape de mise en contact étant suivie par une deuxième étape de séparation entre une deuxième phase liquide et une deuxième phase solide pour ensuite être séchée.

5. Procédé selon l'une quelconque des revendications précédentes, dans lequel ladite phase hydroxyapatite solide est préalablement lavée à l'eau avant d'être séchée.

6. Procédé selon l'une quelconque des revendications précédentes, qu'il comprend, préalablement à la première étape de mise en contact avec ledit liquide d'extraction, une étape de dégraissage et de décharnage dudit matériau osseux.

7. Procédé selon l'une quelconque des revendications précédentes, **caractérisé en ce que**, ultérieurement à ladite étape de séchage, il comprend une étape de stérilisation de ladite phase hydroxyapatite solide sèche.

8. Procédé selon l'une quelconque des revendications précédentes, dans lequel ledit matériau osseux est d'origine naturelle animale.

## Patentansprüche

1. Verfahren zur Herstellung eines Knochenregenerierungsmaterials, umfassend:
- ein In-Kontakt-Bringen eines Knochenmaterials, das Hydroxylapatit und organische Substanzen enthält, mit einer Extraktionsflüssigkeit, das zu einer flüssigen ersten Phase, die die organischen Substanzen und gegebenenfalls aus dem Knochenmaterial extrahierte Unreinheiten enthält, und einer festen zweiten Phase des Hydroxylapatits, die das Hydroxylapatit enthält, führt und
- eine Trennung zwischen der flüssigen ersten Phase und der festen zweiten Phase des Hydroxylapatits,
**dadurch gekennzeichnet, dass** die Extraktionsflüssigkeit eine wässrige Extraktionslösung ist, die auf eine Temperatur zwischen 150 °C und 300 °C und einen Druck zwischen 1500 kPa und 3500 kPa gebracht wird, und dadurch, dass die getrennte feste Phase des Hydroxylapatits das Knochenregenerierungsmaterial bildet.

2. Verfahren nach Anspruch 1, weiter einen zusätzlichen Trocknungsschritt der festen Phase des Hydroxylapatits umfassend, um eine Phase des trockenen festen Hydroxylapatits zu erhalten.

3. Verfahren nach Anspruch 1 oder 2, wobei die wässrige Extraktionslösung Wasser oder eine konzentrierte wässrige Lösung mit basischem pH ist, die eine starke Base mit einer Konzentration zwischen 0,5 und 1 N, vorzugsweise zwischen 0,6 und 0,9 N umfasst.

4. Verfahren nach einem der vorstehenden Ansprüche, **dadurch gekennzeichnet, dass** es nach dem ersten Trennungsschritt und vor dem Trocknungsschritt der festen Phase des Hydroxylapatits einen zweiten Schritt des In-Kontakt-Bringens der festen Phase des Hydroxylapatits mit einer verdünnten wässrigen Lösung mit basischem pH bei einer Temperatur zwischen 150 °C und 300°C und bei einem Druck zwischen 500 kPa und 1000 kPa umfasst, wobei die verdünnte wässrige Lösung mit basischem pH eine starke Base mit einer Konzentration zwischen 0,05 und 0,4 N, vorzugweise zwischen 0,1 und 0,3 N umfasst, wobei der zweite Schritt des In-Kontakt-Bringens von einem zweiten Trennungsschritt zwischen einer flüssigen zweiten Phase und einer festen zweiten Phase, damit diese anschließend getrocknet wird, gefolgt wird.

5. Verfahren nach einem der vorstehenden Ansprüche, wobei die feste Phase des Hydroxylapatits vor der Trocknung vorab mit Wasser ausgewaschen wird.

6. Verfahren nach einem der vorstehenden Ansprüche, dass es vor dem ersten Schritt des In-Kontakt-Bringens mit der Extraktionsflüssigkeit einen Entfettungs- und Fleischentfernungsschritt des Knochenmaterials umfasst.

7. Verfahren nach einem der vorstehenden Ansprüche, **dadurch gekennzeichnet, dass** es im Anschluss an den Trocknungsschritt einen Sterilisierungsschritt der Phase des trockenen festen Hydroxylapatits umfasst.

8. Verfahren nach einem der vorstehenden Ansprüche, **dadurch gekennzeichnet, dass** das Knochenmaterial natürlichen tierischen Ursprungs ist.

## Claims

1. Method for manufacturing a bone regeneration material comprising:
- a putting into contact of a bone material containing hydroxyapatite and organic substances with an extraction liquid that gives rise to a first liquid phase, containing said organic substances and possibly impurities extracted from said bone material, and a second solid hydroxyapatite phase, containing said hydroxyapatite, and
- a separation between said liquid phase and said solid hydroxyapatite phase, **characterised in that** said extraction liquid is an extraction aqueous solution brought to a temperature comprised between 150°C and 300°C and to a pressure comprised between 1500 kPa and 3500 kPa, and **in that** said separated solid hydroxyapatite phase forms said bone regeneration material.

2. Method according to claim 1, further comprising an additional step of drying said solid hydroxyapatite phase to obtain a dry, solid hydroxyapatite phase.

3. Method according to claim 1 or 2, wherein said extraction aqueous solution is water or an aqueous solution with a concentrated basic pH comprising a strong base at a concentration comprised between 0.5 and 1N, preferably between 0.6 and 0.9N.

4. Method according to any one of the preceding claims, **characterised in that**, after said first step of separating and before said step of drying said solid hydroxyapatite phase, it comprises a second step of putting into contact with a diluted aqueous solution with a basic pH of said solid hydroxyapatite phase at a temperature comprised between 150°C and 300°C and at a pressure comprised between 500 kPa and 1000 kPa, said diluted aqueous solution with a basic pH comprising a strong base at a concentration comprised between 0.05N and 0.4N, preferably between 0.1 and 0.3N, said second putting into contact step being followed by a second separation step between a second liquid phase and a second solid phase to then be dried.

5. Method according to any one of the preceding claims, wherein said solid hydroxyapatite phase is washed beforehand with water before being dried.

6. Method according to any one of the preceding claims, that it comprises, prior to the first step of putting into contact with said extraction liquid, a step of grease removal and defleshing said bone material.

7. Method according to any one of the preceding claims, **characterised in that**, subsequent to said drying step, it comprises a step of sterilising said dry, solid hydroxyapatite phase.

8. Method according to any one of the preceding claims, wherein said bone material is of natural animal origin.
